# EUROPEAN PATENT SPECIFICATION

(11) **EP 3 747 442 B1**
(45) Date of publication and mention of the grant of the patent: **10.07.2024**
(21) Application number: 19747763.1
(22) Date of filing: 30.01.2019
(51) Int. Cl.: A61K 9/00, A61K 47/10, A61K 47/14, A61K 47/20, A61K 47/22, A61K 47/44, A61K 31/565, A61P 5/32

(54) **METHOD FOR PRODUCING PREPARATION FOR INJECTION**
VERFAHREN ZUR HERSTELLUNG EINES INJEKTIONSPRÄPARATS
MÉTHODE DE PRODUCTION DE PRÉPARATION POUR INJECTION

(30) Priority: 31.01.2018 JP 2018015311
(43) Date of publication of application: 09.12.2020
(73) Proprietor: FUJIFILM Corporation, Tokyo 106-8620 (JP)
(72) Inventor: SHIONOZAKI, Nobuhiro, Ashigarakami-gun, Kanagawa 258-8577 (JP); SENGA, Kyoko, Ashigarakami-gun, Kanagawa 258-8577 (JP)
(74) Representative: Klunker IP Patentanwälte PartG mbB
(86) International application number: PCT/JP2019/003251
(87) International publication number: WO 2019/151353

(56) References cited:
- WO-A1-2017/208847
- WO-A2-03/006063
- CN-A- 1 444 925
- JP-A- 2004 521 151
- JP-A- 2005 179 200
- JP-A- 2011 190 275
- JP-B2- 4 295 087
- US-A1- 2013 267 489
- ANONYMOUS: "Benzyl benzoate", 2017, pages 1 - 4, XP055781320, Retrieved from the Internet <URL:https://www.chemicalbook.com/ProductChemicalPropertiescb9152283_EN.htm> [retrieved on 20210302]
- THARP I D ET AL: "Production of Benzyl Benzoate", INDUSTRIAL AND ENGINEERING CHEMISTRY, 1 October 1947 (1947-10-01), XP055781355, Retrieved from the Internet <URL:https://pubs.acs.org/doi/pdf/10.1021/ie50454a011> [retrieved on 20210302]

## Description

### BACKGROUND OF THE INVENTION

### 1. Field of the Invention

The present disclosure relates to a method for manufacturing a preparation for injection.

### 2. Description of the Related Art

Fulvestrant (7α-[9-(4,4,5,5,5-pentafluoropentylsulfinyl)nonyl]estra-1,3,5(10)-triene-3,17β-diol) is an estrogen receptor antagonist, and is marketed by AstraZeneca K.K. as an intramuscular injection preparation under the trade name FASLODEX (registered trademark). FASLODEX contains fulvestrant at 50 mg/mL, which is a declared amount, and ethanol, benzyl alcohol, benzyl benzoate, and castor oil as additives, and is supplied in the form of a 5 mL pre-filled syringe.

In recent years, there have been various reports regarding pharmaceutical preparations containing fulvestrant.

For example, Japanese Patent No. 4295087 discloses a pharmaceutical preparation suited for intramuscular administration containing fulvestrant, a ricinoleate additive, a pharmaceutically acceptable non-aqueous ester solvent, a pharmaceutically acceptable alcohol, and an antioxidant selected from a specific group.

Japanese Patent Publication No. 2011-190275 A, International Patent Publication No. 2017/208847 A1 and US Patent Publication No. 2013/267489 A1 disclose injectable formulations comprising fulvestrant and benzyl benzoate. Further, it is known from "Benzyl benzoate", 2017, pages 1-4, XP055781320, retrieved from the internet: URL: https://www.chemicalbook.com/ChemicalProductProperty_EN_CB9152283 .htm that commercial benzyl benzoate is purified before being marketed.

### SUMMARY OF THE INVENTION

It is known that fulvestrant is sulfonated by oxidation and generates an analog called fulvestrant sulfone.

Japanese Patent No. 4295087 discloses the suppression of increase in fulvestrant sulfone during the storage of pharmaceutical preparations by using various antioxidants.

However, according to the study of the inventors of the present invention, it has been found that sometimes fulvestrant sulfone increases at the time of formulating a preparation for injection. The increase in fulvestrant sulfone at the time of formulating a preparation is not mentioned in Japanese Patent No. 4295087.

The increase in fulvestrant sulfone frequently occurs particularly at the time of formulating a preparation for injection containing fulvestrant by using synthetic benzyl benzoate. Therefore, presumably, the oxidation action of impurities such as a trace of peroxide mixed in the synthetic benzyl benzoate may be involved in the generation of fulvestrant sulfone at the time of formulating a preparation for injection.

Therefore, the inventors of the present invention have found a technique of suppressing the increase in fulvestrant sulfone by carrying out steps of performing a specific treatment on benzyl benzoate so as to cancel the oxidation action expressed by impurities such as peroxide mixed in the synthetic benzyl benzoate and then mixing the treated benzyl benzoate with fulvestrant.

In the present specification, synthetic benzyl benzoate refers to a product synthesized by a known method, wherein the quantitative value of benzyl benzoate is equal to or greater than 98.0%.

An object to be achieved by an embodiment of the present invention is set in consideration of the circumstances described above, and is to provide a method for manufacturing a preparation for injection, which suppresses an increase in fulvestrant sulfone at the time of formulating the preparation.

Specific means for achieving the above object include the following embodiments.
[1] A method for manufacturing a preparation for injection, including a first step of performing at least one treatment selected from the group consisting of a treatment of mixing benzyl benzoate with an antioxidant, a treatment of heating benzyl benzoate, and a treatment of purifying benzyl benzoate: and
   a second step of mixing the benzyl benzoate treated by the first step with fulvestrant, wherein the antioxidant in the treatment of mixing benzyl benzoate with an antioxidant is at least one kind of compound selected from the group consisting of ascorbic acid, ascorbic acid palmitate, thiourea, α-tocopherol, δ-tocopherol, α-thioglycerin, cysteine hydrochloride, dihydrolipoic acid, dibutylhydroxytoluene, and propyl gallate, wherein, in the treatment of heating benzyl benzoate, benzyl benzoate is heated to 40°C to 150°C in a nitrogen atmosphere, and wherein, in the treatment of purifying benzyl benzoate, benzyl benzoate is purified using a solid phase column.
[2] The method for manufacturing a preparation for injection described in [1], in which the antioxidant in the treatment of mixing benzyl benzoate with an antioxidant is ascorbic acid.
[3] The method for manufacturing a preparation for injection described in any one of [1] to [2], in which an amount of the antioxidant in the treatment of mixing benzyl benzoate with an antioxidant is 0.1% by mass to 2% by mass with respect to an amount of benzyl benzoate.
[4] The method for manufacturing a preparation for injection described in any one of [1] to [3], in which the treatment of mixing benzyl benzoate with an antioxidant is a treatment of mixing benzyl benzoate with an antioxidant and a medically acceptable solvent.
[5] The method for manufacturing a preparation for injection described in [4], in which the medically acceptable solvent is an alcohol.
[6] The method for manufacturing a preparation for injection described in [5], in which the alcohol is at least one alcohol selected from the group consisting of ethanol, benzyl alcohol, propylene glycol, polyethylene glycol, and glycofurol.
[7] The method for manufacturing a preparation for injection described in [5] or [6], in which the alcohol is at least one alcohol selected from the group consisting of ethanol, benzyl alcohol, and propylene glycol.

According to the present invention, the above method for manufacturing a preparation for injection suppresses the increase in fulvestrant sulfone at the time of formulating a preparation.

### DESCRIPTION OF THE PREFERRED EMBODIMENTS

Hereinafter, embodiments of a method for manufacturing a preparation for injection to which the present invention is applied will be described. The scope of protection of the invention is limited by the appended claims. It is not at all limited by the following embodiments, and can be embodied by being appropriately modified as long as the modifications fall within the scope of the claims.

In the present specification, a range of numerical values described using "to" means a range including numerical values described before and after "to" as a minimum value and a maximum value, respectively.

Regarding the ranges of numerical values described stepwise in the present specification, the upper limit or the lower limit described in a certain range of numerical values may be replaced with the upper limit or the lower limit of another range of numerical values described stepwise.

In addition, in the ranges of numerical values described in this specification, the upper limit or the lower limit described in a certain numerical range may be replaced with the value shown in the Examples.

In the present specification, in a case where a preparation for injection contains a plurality of substances corresponding to each component, unless otherwise specified, the amount of each component in the preparation for injection means the total amount of the plurality of substances in the preparation for injection.

In the present specification, a combination of two or more preferred embodiments is a more preferred embodiment.

In the present specification, the term "step" includes not only an independent step but also a step which is not clearly distinguished from another step as long as the intended purpose of the step is achieved.

### <Method for manufacturing preparation for injection>

The method for manufacturing a preparation for injection of the present disclosure includes a first step of performing at least one treatment selected from the group consisting of a treatment of mixing benzyl benzoate with an antioxidant, a treatment of heating benzyl benzoate, and a treatment of purifying benzyl benzoate (hereinafter, simply referred to as "first step" as well) and a second step of mixing the benzyl benzoate treated by the first step with fulvestrant (hereinafter, simply referred to as "second step" as well).

Hereinafter, each step will be described.

### [First step]

In the first step, at least one treatment is performed which is selected from the group consisting of a treatment of mixing benzyl benzoate with an antioxidant (hereinafter, referred to as "mixing treatment" as well), a treatment of heating benzyl benzoate (hereinafter, referred to as "heating treatment" as well), and a treatment of purifying benzyl benzoate (hereinafter, referred to as "purification treatment" as well).

By any of the above treatments, it is possible to obtain a benzyl benzoate-containing treated product which contains benzyl benzoate and hardly generates fulvestrant sulfone.

### [Benzyl benzoate]

The benzyl benzoate used in the first step is preferably synthetic benzyl benzoate.

Specifically, commercial benzyl benzoate such as Benzyl Benzoate from Merck KGaA, Benzyl Benzoate from PanReac AppliChem, Benzyl Benzoate from Spectrum, Benzyl Benzoate from Wako Pure Chemical Industries, Ltd., Benzyl Benzoate from TOKYO CHEMICAL INDUSTRY CO., LTD., and Benzyl Benzoate from Sigma-Aldrich Co. LLC. are suitable.

The synthetic benzyl benzoate has a possibility that the content of impurities such as peroxide mixed in the benzyl benzoate may differ between manufacturing lots. However, due to the nature of peroxide, it is difficult to measure the peroxide mixed in the benzyl benzoate.

Furthermore, after the benzyl benzoate is mixed with fulvestrant, it is difficult to remove fulvestrant sulfone generated by the impurities such as peroxide mixed in the benzyl benzoate. Therefore, it is considered that there is a big problem in additionally performing a step of removing the generated fulvestrant sulfone in the process of manufacturing a preparation for injection containing fulvestrant.

Accordingly, regardless of the difference in the content of impurities such as peroxide between manufacturing lots of benzyl benzoate and regardless of the type of benzyl benzoate used, by performing the treatment in the first step as in the method for manufacturing a preparation for injection of the present disclosure, it is possible to stably suppress an increase in fulvestrant sulfone.

### [Treatment of mixing benzyl benzoate with antioxidant: mixing treatment]

In the mixing treatment, benzyl benzoate is mixed with an antioxidant.

By performing the mixing treatment it is possible to obtain a benzyl benzoate-containing treated product in which oxidation action on fulvestrant is canceled by the action of the antioxidant.

### (Antioxidant)

From the viewpoint of maintaining the ability to prevent the oxidation of fulvestrant in the formulated preparation for injection, the antioxidant is at least one compound selected from the group consisting of ascorbic acid, ascorbic acid palmitate, thiourea, α-tocopherol. δ-tocopherol, α-thioglycerin. cysteine hydrochloride, dihydrolipoic acid, dibutylhydroxytoluene, and propyl gallate.

Particularly, as the antioxidant to be mixed with benzyl benzoate, ascorbic acid is preferable because this compound has been used in preparations for injection.

In the mixing treatment, the amount of the antioxidant to be mixed with benzyl benzoate with respect to the amount of the benzyl benzoate is preferably 0.01% by mass to 20% by mass, more preferably 0.01% by mass to 5% by mass, and even more preferably 0.1% by mass to 2% by mass.

One kind of antioxidant may be used singly, or two or more kinds of antioxidants may be used by being mixed together.

### (Medically acceptable solvent)

The aforementioned antioxidant expresses an antioxidation ability as long as it is partially dissolved in benzyl benzoate. However, from the viewpoint of further enhancing the expression of the antioxidation ability, in the mixing treatment, it is preferable to use a solvent for improving the solubility of the antioxidant.

That is, the mixing treatment may be a treatment of mixing benzyl benzoate with an antioxidant and a medically acceptable solvent.

The solvent used herein is a medically acceptable solvent other than benzyl benzoate, and may be determined according to the solubility of the antioxidant to be used.

The medically acceptable solvent used in the mixing treatment is not particularly limited, but is preferably an alcohol which can easily dissolve the antioxidant and can improve the solubility of fulvestrant.

One kind of alcohol may be used singly, or two or more kinds of alcohols may be used by being mixed together.

Specific examples of the alcohol include at least one alcohol selected from the group consisting of ethanol, benzyl alcohol, propylene glycol, polyethylene glycol, and glycofurol. Among these, at least one alcohol selected from the group consisting of ethanol, benzyl alcohol, and propylene glycol is more preferable.

Ethanol is not particularly limited as long as it has quality that meets the standards of pharmacopeias (those described in pharmacopeias in Japan, the United States, Europe, and other countries).

Examples of the ethanol in the preparation for injection of the present disclosure include the ethanol described in the Japanese Pharmacopoeia (containing ethanol at 95.1% by volume to 96.9% by volume at 15°C) and absolute ethanol (containing ethanol at a proportion equal to or higher than 99.5% by volume at 15°C).

### (Combination of antioxidant and medically acceptable solvent)

Hereinafter, preferred combinations of an antioxidant and a medically acceptable solvent will be described.

In a case where ascorbic acid is used as an antioxidant, as a medically acceptable solvent, it is preferable to combine an alcohol with the antioxidant.

The alcohol is preferably at least one alcohol selected from the group consisting of ethanol, benzyl alcohol, propylene glycol, polyethylene glycol, and glycofurol. Among these, at least one alcohol selected from the group consisting of ethanol, benzyl alcohol, and propylene glycol is more preferable.

In a case where ascorbic acid palmitate, thiourea, α-tocopherol, δ-tocopherol, α-thioglycerin, cysteine hydrochloride, dihydrolipoic acid, dibutylhydroxytoluene, or propyl gallate is used as an antioxidant, as a medically acceptable solvent, it is also preferable to combine an alcohol with the antioxidant.

The alcohol is preferably at least one alcohol selected from the group consisting of ethanol. benzyl alcohol, propylene glycol, polyethylene glycol, and glycofurol. Among these, at least one alcohol selected from the group consisting of ethanol, benzyl alcohol, and propylene glycol is more preferable.

The medically acceptable solvent used in the mixing treatment may be used in such an amount that the content rate of the medically acceptable solvent in the preparation for injection falls into the range which will be described later.

### (Mixing)

In the mixing treatment, benzyl benzoate, an antioxidant, and a medically acceptable solvent used as needed may be simply mixed together. All of these components may be mixed together at once, or these components may be mixed together in several divided portions.

The mixing method is not particularly limited, and examples thereof include mixing by stirring.

### -Mixing time-

The mixing time in the mixing treatment is preferably set such that the antioxidation ability of the antioxidant is sufficiently expressed.

In this step, "mixing time" refers to the time from the start of mixing of benzyl benzoate with an antioxidant to the start of mixing of fulvestrant in the second step as the next step.

The mixing time is not particularly limited, and may be appropriately determined according to the type and amount of benzyl benzoate, the mixing temperature, the atmosphere at the time of mixing, the manufacturing scale, the mixing efficiency, the manufacturing efficiency, and the like.

For example, the mixing time may be 1 minute to 1 day (that is, 24 hours) or 10 minutes to 12 hours.

### -Temperature at the time of mixing-

The temperature condition at the time of mixing is not particularly limited, and can be appropriately set, for example, according to the amount and solubility of the antioxidant to be mixed, and the like.

For example, the mixing treatment is preferably performed under the condition of an atmospheric temperature of 4°C to 70°C, and more preferably performed under the condition of an atmospheric temperature of 15°C to 60°C

### -Atmosphere at the time of mixing-

The benzyl benzoate and the antioxidant can be mixed together in the air, or may be mixed together in a nitrogen atmosphere. It is desirable that the benzyl benzoate and the antioxidant are mixed together in a nitrogen atmosphere.

[Treatment of heating benzyl benzoate: heating treatment]

In the heating treatment, the benzyl benzoate is heated.

By heating the benzyl benzoate, the oxidation action of impurities contained in the synthetic benzyl benzoate is reduced (specifically, the decomposition of peroxide as an impurity results in the reduction of the antioxidation action). As a result, by the heating treatment, a benzyl benzoate-containing treated product in which the oxidation action on fulvestrant is canceled is obtained.

### (Heating)

### -Heating temperature-

in the heating treatment, the benzyl benzoate is heated. From the viewpoint of effectively reducing the oxidation action of impurities, the benzyl benzoate is heated to 40°C to 150°C.

### -Heating time-

The heating time in the heating treatment is not particularly limited, and may be determined according to the heating temperature, the manufacturing scale, the heating efficiency, and the like.

For example, from the viewpoint of effectively reducing the oxidation action of impurities, the benzyl benzoate is preferably heated for 1 minute to 1 week, and more preferably heated for 1 minute to 1 day (that is, 24 hours).

### -Atmosphere at the time of heating-

In the heating treatment, the benzyl benzoate is heated in a nitrogen atmosphere.

### -Heating unit-

Examples of the heating unit used in the heating treatment include an autoclave, microwave heating, a hot water bath, an oil bath, a hot stirrer, and the like.

The heating treatment for benzyl benzoate may be performed only on benzyl benzoate or on a mixture of benzyl benzoate and a medically acceptable solvent.

As the medically acceptable solvent used in the heating treatment, a medically acceptable solvent used in the aforementioned mixing treatment is used, and preferred embodiments thereof are also the same.

### [Treatment of purifying benzyl benzoate: purification treatment]

In the purification treatment, the benzyl benzoate is purified.

By purifying the benzyl benzoate, impurities contained in the synthetic benzyl benzoate are removed or reduced. As a result, by the purification treatment, a benzyl benzoate-containing treated product in which the oxidation action on fulvestrant is canceled is obtained.

### (Purification treatment)

### -Purification unit-

Examples of purification techniques include purification using a column, purification by distillation, purification by extraction, and the like. Among these, from the view point of high performance of removing or reducing impurities contained in the synthetic benzyl benzoate, purification using a solid phase column is the technique according to the invention.

### [Second step]

In the second step, the benzyl benzoate treated by the first step is mixed with fulvestrant.

### [Fulvestrant]

Fulvestrant used in the second step is an active component of the preparation for injection obtained by the method for manufacturing a preparation for injection of the present disclosure.

Fulvestrant (7α-[9-(4,4,5,5,5-pentafluoropentylsulfinyl)nonyl]estra-1,3,5(10)-triene-3,17β-diol) is an estrogen receptor antagonist and is known as a drug for treating breast cancer and the like.

The fulvestrant used in the second step may be used in such an amount that the content rate of fulvestrant in the preparation for injection falls into the range which will be described later.

### (Mixing)

By the mixing in the second step, the benzyl benzoate-containing treated product obtained by the first step may be simply mixed with fulvestrant. All of these components may be mixed together at once, or these components may be mixed together in several divided portions.

The mixing method is not particularly limited, and examples thereof include mixing by stirring.

The mixing time in the second step is preferably a time sufficient for the fulvestrant to dissolve.

In this step, "mixing time" refers to the time from the start of mixing of the benzyl benzoate-containing treated product obtained by the first step with fulvestrant to the stopping of the mixing method such as stirring.

The mixing time may be appropriately determined according to the amount of fulvestrant, the mixing temperature, the atmosphere at the time of mixing, the manufacturing scale, the mixing efficiency, the manufacturing efficiency, and the like.

For example, the mixing time may be 1 minute to 1 day (that is, 24 hours) or 10 minutes to 12 hours.

The temperature condition at the time of mixing in the second step is not particularly limited, and can be appropriately set according to, for example, the type, amount, and solubility of components to be mixed together, and the like.

Generally, the mixing treatment is preferably performed under the condition of an atmospheric temperature of 4°C to 70°C, and more preferably performed under the condition of an atmospheric temperature of 15°C to 60°C.

### -Atmosphere at the time of mixing-

The mixing in the second step can be performed in the air, but may be performed in a nitrogen atmosphere. It is desirable that the mixing is performed in a nitrogen atmosphere.

### [Other steps]

The method for manufacturing a preparation for injection of the present disclosure may include other steps in addition to the first step and the second step.

Examples of those other steps include a step of adding a medically acceptable solvent to benzyl benzoate and mixing these together (hereinafter, referred to as "third step" as well), a step of sterilizing the preparation for injection, a step of filling a container with the preparation for injection, and the like.

### [Third step]

In the method for manufacturing a preparation for injection of the present disclosure, in a case where a medically acceptable solvent is not used in the first step or in a case where a small amount of a medically acceptable solvent is used in the first step, a step of mixing a medically acceptable solvent with the benzyl benzoate treated by the first step (that is, the third step) may be performed between the first step and the second step, during the second step. or after the second step.

In a case where the third step is performed, according to the timing of performing the third step, at least any one component among the benzyl benzoate, the fulvestrant, and the antioxidant in the preparation for injection undergoes concentration adjustment or the like.

Herein, performing the third step during the second step means that a medically acceptable solvent is also mixed together at the time of mixing fulvestrant with the benzyl benzoate-containing treated product which is obtained by the first step and contains benzyl benzoate.

Examples of the medically acceptable solvent used in the third step include an alcohol, vegetable oil, and a non-aqueous ester compound except for benzyl benzoate.

In the present disclosure, "vegetable oil" means an oil or fat extracted from plant seeds, fruit, or the like. As the vegetable oil, a vegetable oil that has been used as a pharmaceutical additive is preferable.

In the present disclosure, "non-aqueous" specifically means that the solubility in water at 25°C is less than 1% by mass.

Examples of the alcohol include the alcohols described in the section of the mixing treatment in the first step, and preferred examples thereof are also the same.

Specific examples of the vegetable oil include castor oil, sesame oil, peanut oil. soybean oil, camellia oil, corn oil, cottonseed oil, olive oil, safflower oil, rapeseed oil, and the like.

Examples of the non-aqueous ester compound include ethyl oleate, isopropyl myristate, diethyl sebacate, and the like.

Furthermore, in the method for manufacturing a preparation for injection of the present disclosure, the third step is preferably a step of mixing castor oil with a mixture containing benzyl benzoate and fulvestrant after the second step.

Particularly, by performing the step of mixing castor oil with a mixture containing benzyl benzoate and fulvestrant, it is possible to improve the solubility of the fulvestrant in the preparation for injection and to improve the sustained release properties of the fulvestrant after injection.

In the third step, in addition to castor oil, a vegetable oil different from castor oil may be used. That is, in the third step, a mixture of castor oil and a vegetable oil different from castor oil may be used.

As the vegetable oil different from castor oil, from the viewpoint of the solubility of the fulvestrant and further improving the sustained release properties of the fulvestrant after injection, at least one kind of vegetable oil is preferable which is selected from the group consisting of sesame oil, peanut oil, soybean oil, camellia oil, corn oil, cottonseed oil, olive oil, safflower oil, rapeseed oil, and fatty acid triglyceride constituted with a fatty acid having 6 to 12 carbon atoms on average (that is, medium-chain fatty acid triglyceride).

In addition, as the vegetable oil different from castor oil, at least one kind of vegetable oil selected from the group consisting of sesame oil, peanut oil, soybean oil, camellia oil, and corn oil is preferable because these oils have been used in injections given by intramuscular injection in Japan and foreign countries; and at least one kind of vegetable oil selected from sesame oil and peanut oil is more preferable because these oils have been used in injections given by intramuscular injection in Japan.

In a case where the aforementioned mixture is used in the third step, from the viewpoint of improving the sustained release properties of the fulvestrant after injection, the type and content rate of the vegetable oil different from castor oil is preferably set in consideration of the solubility of the fulvestrant.

Specifically, in the mixture, the content rate of the vegetable oil different from castor oil is preferably equal to or lower than 20% by mass, and more preferably equal to or lower than 10% by mass.

The mixing method in the third step is not particularly limited, and examples thereof include mixing by stirring.

In the third step, the components to be mixed together may be mixed at once. Alternatively, for example, while the solution containing fulvestrant is being stirred, a medically acceptable solvent may be slowly added thereto and mixed together.

The temperature condition at the time of mixing in the third step is not particularly limited.

In the third step, usually, it is preferable to perform mixing under the condition of an atmospheric temperature of 15°C to 60°C.

According to the method for manufacturing a preparation for injection of the present disclosure, the increase in fulvestrant sulfone at the time of formulating the preparation is suppressed through the above steps.

Specifically, through the above steps, the rate of increase in fulvestrant sulfone can be equal to or lower than 200%, and more preferably equal to or lower than 100%.

The rate of increase in fulvestrant sulfone is calculated by comparing fulvestrant sulfone in the fulvestrant used in the second step with fulvestrant sulfone contained in the formulated preparation for injection. A specific calculation method will be described in the Examples.

### <Preparation for injection>

By the method for manufacturing a preparation for injection of the present disclosure, a preparation for injection in which the increase in fulvestrant sulfone at the time of formulating the preparation is suppressed can be obtained.

Because the increase in fulvestrant sulfone at the time of formulating the preparation is suppressed, the preparation for injection obtained by the method for manufacturing a preparation for injection of the present disclosure can be stored for a long period of time.

It is preferable that the obtained preparation for injection has the following composition.

For example, from the viewpoint of improving the solubility of fulvestrant, the content rate of benzyl benzoate in the preparation for injection with respect to the total mass of the preparation for injection is preferably equal to or higher than 5% by mass and equal to or lower than 20% by mass, and more preferably equal to or higher than 10% by mass and equal to or lower than 20% by mass.

The content rate of the fulvestrant in the preparation for injection may be equal to or higher than 2.0% by mass, for example, with respect to the total mass of the preparation for injection. From the viewpoint of dosage, the content rate of the fulvestrant with respect to the total mass of the preparation for injection is preferably equal to or higher than 4.5% by mass, and more preferably equal to or higher than 5.0% by mass.

The upper limit of the content rate of the fulvestrant in the preparation for injection is not particularly limited. For example, from the viewpoint of the solubility of the fulvestrant, the upper limit with respect to the total mass of the preparation for injection is preferably equal to or lower than 10% by mass, more preferably equal to or lower than 7.0% by mass, and even more preferably equal to or lower than 5.5% by mass.

For example, from the viewpoint of suppressing the generation and increase of fulvestrant sulfone, the content rate of the antioxidant in the preparation for injection with respect to the total mass of the preparation for injection is preferably equal to or higher than 0.001% by mass and equal to or lower than 1% by mass, more preferably equal to or higher than 0.005% by mass and equal to or lower than 0.5% by mass, and even more preferably equal to or higher than 0.01% by mass and equal to or lower than 0.15% by mass.

For example, from the viewpoint of the solubility of the fulvestrant and the antioxidant, the content rate of the medically acceptable solvent (except for benzyl benzoate) in the preparation for injection with respect to the total mass of the preparation for injection is preferably equal to or higher than 60% by mass and equal to or lower than 90% by mass, more preferably equal to or higher than 65% by mass and equal to or lower than 90% by mass, and even more preferably equal to or higher than 70% by mass and equal to or lower than 90% by mass.

The content rate of the alcohol in the medically acceptable solvent is preferably as follows.

That is, for example, from the viewpoint of the solubility of the fulvestrant and the antioxidant, the content rate of the alcohol in the preparation for injection with respect to the total mass of the preparation for injection is preferably equal to or higher than 5% by mass and equal to or lower than 40% by mass, more preferably equal to or higher than 7% by mass and equal to or lower than 35% by mass, and even more preferably equal to or higher than 10% by mass and equal to or lower than 30% by mass.

The content rate of castor oil in the medically acceptable solvent is preferably as follows.

That is, for example, from the viewpoint of the solubility of the fulvestrant and the antioxidant, the content rate of the castor oil in the preparation for injection with respect to the total mass of the preparation for injection is preferably equal to or higher than 35% by mass and equal to or lower than 80% by mass, more preferably equal to or higher than 40% by mass and equal to or lower than 75% by mass, and even more preferably equal to or higher than 45% by mass and equal to or lower than 70% by mass.

From the viewpoint of suppressing turbidity that can occur in a case where the preparation for injection is stored at a low temperature, it is preferable that water is not intentionally mixed with the preparation for injection.

"Low temperature" mentioned herein generally refers to a temperature adopted in a case where a preparation for injection containing fulvestrant as an active component is stored refrigerated. Specifically, "low temperature" means a range of 2°C to 8°C.

In addition, "water is not intentionally mixed" means that except for water, which is inevitably incorporated into the preparation for injection of the present disclosure due to the moisture that the components of the preparation contain at the state of raw materials and is thus added to the preparation, and water which is inevitably incorporated into the preparation for injection due to the absorption of moisture from the atmosphere, water is not mixed with the preparation for injection as far as possible. "Water incorporated into the preparation for injection due to the absorption of moisture from the atmosphere" includes water retained in the preparation for injection during the storage of the preparation for injection.

Specifically, the content rate of water in the preparation for injection with respect to the total mass of the preparation for injection is preferably equal to or lower than 5% by mass, more preferably equal to or lower than 3% by mass, even more preferably equal to or lower than 2% by mass, and particularly preferably equal to or lower than 1% by mass.

If necessary, the preparation for injection may further contain pharmaceutically acceptable additives (hereinafter, referred to as "other additives" as well) in addition to the components described above. In a case where the preparation for injection obtained by the method for manufacturing a preparation for injection of the present disclosure is used for intramuscular injection, it is preferable that the preparation for injection further contains additives suitable for intramuscular injection.

Examples of those other additives include gluconic acid or a salt thereof, acetic acid or a salt thereof, lactic acid or a salt thereof, boric acid or a salt thereof, phosphoric acid or a salt thereof, sulfuric acid or a salt thereof, tartaric acid or a salt thereof, citric acid or a salt thereof, potassium hydroxide, calcium hydroxide, sodium hydroxide, magnesium hydroxide, monoethanolamine, diethanolamine, triethanolamine, trometamol, meglumine, glycine, histidine or a salt thereof, ε-aminocaproic acid, arginine or a salt thereof, alanine, leucine, aspartic acid or a salt thereof, glutamic acid or a salt thereof, arginine or a salt thereof, taurine, sodium edetate, lidocaine or a salt thereof, nicotinamide, chlorobutanol, creatinine, sorbitan sesquioleate, ethyl lactate, tylomesal, polyoxyethylene hydrogenated castor oil, polysorbate 20, polysorbate 80, medium-chain fatty acid triglyceride, and the like.

However, those other additives are not limited to these.

In a case where the preparation for injection contains other additives, the preparation may contain only one kind of those other additives or two or more kinds of those other additives.

In a case where the preparation for injection obtained by the method for manufacturing a preparation for injection of the present disclosure contains other additives, the content rate of those other additives in the preparation for injection with respect to the total mass of the preparation for injection is preferably equal to or lower than 10% by mass, more preferably equal to or lower than 5% by mass, and even more preferably equal to or lower than 3% by mass.

### [Use of preparation for injection]

The preparation for injection obtained by the method for manufacturing a preparation for injection of the present disclosure can be suitably used for intramuscular injection.

Furthermore, because the preparation for injection obtained by the method for manufacturing a preparation for injection of the present disclosure contains fulvestrant as an active component, the preparation can be suitably used for treating breast cancer, uterine myoma, and endometriosis.

### [Container for preparation for injection]

Examples of the container to be filled with the preparation for injection obtained by the method for manufacturing a preparation for injection of the present disclosure include a vial, an ampule, a syringe, and the like.

Among these, as the container to be filled with the preparation for injection, from the viewponit of handleability in the healthcare setting, a syringe is preferable, and a glass syringe is more preferable. That is, as the dosage form of the preparation for injection, a prefilled syringe is preferable which is obtained by filling a syringe with the preparation for injection in advance.

### Examples

Hereinafter, the present invention will be more specifically described with reference to examples. However, as long as the gist of the present invention is maintained, the present invention is not limited to the following examples.

### [Manufacturing of preparation for injection]

### <Example A1>

### (First step: mixing treatment)

Benzyl benzoate (15 parts by mass), 0.05 parts by mass of ascorbic acid, 10 parts by mass of 96% by volume ethanol, and 10 parts by mass of benzyl alcohol were put in a clean glass container including a stirring rod, and mixed together by stirring in the air.

The mixing time was 30 minutes, and the atmospheric temperature at the time of mixing was 25°C.

As the benzyl benzoate, Benzyl Benzoate from Merck KGaA was used (quantitative value (assay) 100.3%, manufacturing lot No. K46888906 551).

### (Second step)

Then, in the air, the benzyl benzoate-containing mixture obtained through the first step (that is, the mixing treatment) was mixed with 5 parts by mass of fulvestrant, thereby obtaining a fulvestrant-containing solution.

The mixing time was 30 minutes, and the atmospheric temperature at the time of mixing was 25°C.

### (Third step)

Subsequently, castor oil was added to the fulvestrant-containing solution such that the total amount thereof was adjusted to 100 parts by mass, and the solution was homogenized by being further stirred, thereby obtaining a preparation for injection of Example A1.

### <Examples A2 to A 13>

Preparations for injection of Examples A2 to A13 were obtained by performing the same operation as that in Example A1, except that the composition of the antioxidant and the solvent used in the first step was changed to the composition shown in the following Table 1.

### <Example A14>

A preparation for injection of Example A14 was obtained by performing the same operation as that in Example A1, except that the composition of the antioxidant and the solvent used in the first step was changed to the composition shown in the following Table 1, and the amount of fulvestrant in the second step was changed to 2 parts by mass.

### <Examples A15 and A 16>

Preparations for injection of Examples A 15 and A16 were obtained by performing the same operation as that in Example A1, except that the mixing condition in the first step was changed to the condition described in the following Table 1.

### <Comparative Example A1>

A preparation for injection of Comparative Example A1 was obtained by performing the same operation as that in Example A1, except that the first step and the second step were changed to the following step a, and a third step was performed after the step a.

### (Step a)

That is, 5 parts by mass of fulvestrant, 15 parts by mass of benzyl benzoate. 0.05 parts by mass of ascorbic acid, 10 parts by mass of 96% by volume ethanol, and 10 parts by mass of benzyl alcohol were put in a clean glass container including a stirring rod, and mixed together by stirring.

The mixing time was 30 minutes, and the atmospheric temperature at the time of mixing was 25°C.

### Comparative Example A2>

A preparation for injection of Comparative Example A2 was obtained by performing the same operation as that in Comparative Example A1, except that ascorbic acid was not used in the step a.

### [Evaluation]

### -Measurement of fulvestrant sulfone-

Each of the preparations for injection of examples and comparative examples obtained as above was weighed (110 µL) and put in a glass vial having a volume of 5 mL (manufactured by NICHIDEN-RIKA GLASS CO., LTD., model: SV-5). Furthermore, by using a transfer pipette, methanol for HPLC was added thereto exactly in an amount of 1 mL, and the resulting solution was stirred using a vortex mixer, thereby obtaining a homogeneous solution.

The obtained solution was filtered using an all plastic syringe and a Millex syringe filter (manufactured by Merck Millipore, pore size: 0.22 µm, material: PVDF), thereby obtaining an evaluation sample 1.

For the preparation for injection of Example A14, the evaluation sample 1 was obtained by the following method.

That is, the preparation for injection of Example A14 was weighed (275 µL) and put in a glass vial having a volume of 5 mL (manufactured by NICHIDEN-RIKA GLASS CO., LTD., model: SV-5). Furthermore, by using a transfer pipette, methanol for HPLC was added thereto exactly in an amount of 1 mL, and the resulting solution was stirred using a vortex mixer, thereby obtaining a homogeneous solution.

The obtained solution was filtered using an all plastic syringe and a Millex syringe filter (manufactured by Merck Millipore, pore size: 0.22 µm, material: PVDF), thereby obtaining an evaluation sample 1.

The obtained evaluation sample 1 was measured by high performance liquid chromatography (HPLC) under the following condition, and the proportion (% by mass) of fulvestrant sulfone was determined.

Furthermore, the proportion (% by mass) of fulvestrant sulfone in the bulk drug of fulvestrant used in examples and comparative examples was determined by preparing an evaluation sample 2.

The evaluation sample 2 was prepared as follows.

That is, 50 mg of the bulk drug of fulvestrant was weighed and put in a glass volumetric flask having a volume of 10 mL, and the volume was increased using methanol for HPLC.

The obtained solution was filtered using an all plastic syringe and a Millex syringe filter (manufactured by Merck Millipore, pore size: 0.22 µm, material: PVDF), thereby obtaining a measurement sample 2.

The obtained evaluation sample 2 was measured by high performance liquid chromatography (HPLC) under the following conditions, and the proportion (% by mass) of fulvestrant sulfone was determined.

### -HPLC condition-

Column: XBridge C8 (product name, particle size: 3.5 µm, column size: 4.6 mm × 150 mm, WATERS)
Mobile phase A: water/acetonitrile/methanol = 41/32/27
Mobile phase B: water/acetonitrile/methanol = 10/49/41
Gradient condition (proportion of mobile phase B): 0% (start) → 0% (25 min) → 100% (55 min) → 100% (65 min) → 0% (66 min) → 0% (70 min, stop)
Detection wavelength: 225 nm
Flow rate: 2.0 mL/min
Column temperature: 40°C

From a proportion X (% by mass) of fulvestrant sulfone in the evaluation sample 1 and a proportion Y (% by mass) of fulvestrant sulfone in the evaluation sample 2, the rate of increase in fulvestrant sulfone was calculated by the following equation. Rate of increase in fulvestrant sulfone (%) = [proportion X (% by mass) - proportion Y (% by mass)]/proportion Y (% by mass) x 100

Then, from the calculated rate of increase in fulvestrant sulfone, the increase in fulvestrant sulfone at the time of formulating the preparation was evaluated based on the following evaluation standard.

### -Evaluation standard-

A: The rate of increase in fulvestrant sulfone is equal to or lower than 100%.
B: The rate of increase in fulvestrant sulfone is higher than 100% and equal to or lower than 150%.
C: The rate of increase in fulvestrant sulfone is higher than 150% and equal to or lower than 200%.
D: The rate of increase in fulvestrant sulfone is higher than 200%.

**[Table 1]**

| | Antioxidant | | Solvent | | | | Mixing condition | | Rate of increase in fulvestrant sulfone [%] | Evaluation |
|---|---|---|---|---|---|---|---|---|---|---|
| | Type | Amount [part by mass] | Type | Amount [part by mass] | Type | Amount [part by mass] | Mixing time [min] | Atmospheric temperature [°C] | | |
| Example A1 | Ascorbic acid | 0.05 | EtOH | 10 | BnOH | 10 | 30 | 25 | 89 | A |
| Example A2 | Ascorbic acid palmitate | 0.05 | EtOH | 10 | BnOH | 10 | 30 | 25 | 95 | A |
| Example A3 | α-Tocopherol | 0.05 | EtOH | 10 | BnOH | 10 | 30 | 25 | 176 | C |
| Example A4 | δ-Tocopherol | 0.05 | EtOH | 10 | BnOH | 10 | 30 | 25 | 190 | C |
| Example A5 | Thiourea | 0.05 | EtOH | 10 | BnOH | 10 | 30 | 25 | -7 | A |
| Example A6 | α-Thioglycerin | 0.05 | EtOH | 10 | BnOH | 10 | 30 | 25 | 52 | A |
| Example A7 | Cysteine hydrochloride | 0.05 | EtOH | 10 | BnOH | 10 | 30 | 25 | 66 | A |
| Example A8 | Dihydrolipoic acid | 0.05 | EtOH | 10 | BnOH | 10 | 30 | 25 | 50 | A |
| Example A9 | Dibutylhydroxytoluene | 0.05 | EtOH | 10 | BnOH | 10 | 30 | 25 | 157 | C |
| Example A 10 | Propyl gallate | 0.05 | EtOH | 10 | BnOH | 10 | 30 | 25 | 177 | C |
| Example A11 | Ascorbic acid | 0.02 | EtOH | 10 | BnOH | 10 | 30 | 25 | 80 | A |
| Example A12 | Ascorbic acid | 0.10 | EtOH | 10 | BnOH | 10 | 30 | 25 | 82 | A |
| Example A 13 | Ascorbic acid | 0.05 | PG | 10 | BnOH | 10 | 30 | 25 | 120 | B |
| Example A14 | Ascorbic acid | 0.05 | PEG400 | 10 | BnOH | 10 | 30 | 25 | 117 | B |
| Example A 15 | Ascorbic acid | 0.05 | EtOH | 10 | BnOH | 10 | 60 | 25 | 166 | C |
| Example A16 | Ascorbic acid | 0.05 | EtOH | 10 | BnOH | 10 | 60 | 40 | 85 | A |
| Comparative Example A1 | Ascorbic acid | 0.05 | EtOH | 10 | BnOH | 10 | 30 | 25 | 246 | D |
| Comparative Example A2 | - | - | EtOH | 10 | BnOH | 10 | 30 | 25 | 236 | D |

In Table 1, "Ethanol" is described as "EtOH", "Propylene glycol" is described as "PG", polyethylene glycol (Wako Pure Chemical Industries, Ltd., polyethylene glycol 400) is described as "PEG400", and "benzyl alcohol" is described as "BnOH".

In Table 1, the mixing condition described for Examples A1 to A16 is the mixing condition in the first step, and the mixing condition described for Comparative Examples A1 and A2 is the mixing condition in the step a.

As is evident from Table 1, in the preparations for injection of Examples A1 to A16, the rate of increase in fulvestrant sulfone is reduced further than in the preparations for injection of Comparative Examples A1 and A2, and the increase in fulvestrant sulfone at the time of formulating the preparations for injection of Examples A1 to A16 is suppressed.

In Examples A1 to A16, the mixing treatment in the first step was performed in the air. However, in a case where the mixing treatment was performed in a nitrogen atmosphere, the rate of increase in fulvestrant sulfone tended to be further reduced.

### <Example B1>

### (First step: heating treatment)

Benzyl benzoate was dispensed into a vial under a nitrogen atmosphere, and the vial was stopped with an aluminum seal and heated to 121 °C in an autoclave for 20 minutes.

As the benzyl benzoate, Benzyl Benzoate from Merck KGaA was used (quantitative value (assay) 100.3%, manufacturing lot No. K46888906 551).

### (Second step)

The benzyl benzoate (15 parts by mass) obtained through the first step (that is, heating treatment), 10 parts by mass of 96% by volume ethanol, 10 parts by mass of benzyl alcohol, and 5 parts by mass of fulvestrant were put in a clean glass container including a stirring rod, and mixed together by stirring, thereby obtaining a fulvestrant-containing solution.

The mixing time was 30 minutes, and the atmospheric temperature at the time of mixing was 25°C.

### (Third step)

Subsequently, castor oil was added to the fulvestrant-containing solution such that the total amount thereof was adjusted to 100 parts by mass, and the solution was homogenized by being further stirred, thereby obtaining a preparation for injection of Example B1.

For the obtained preparation for injection of Example B1, the rate of increase in fulvestrant sulfone was measured and evaluated by the same method as that described above.

As a result, it has been found that in the preparation for injection of Example B1, the rate of increase in fulvestrant sulfone was 147% which is lower than the rate of increase in fulvestrant sulfone in the preparation for injection of Comparative Example A2, and the increase in fulvestrant sulfone at the time of formulating the preparation for injection of Examaple B1 is suppressed.

### <Example C1>

### (First step: purification treatment)

Benzyl benzoate was purified by passing through a solid phase column (InertSep AL-N, GL Sciences Inc.).

As the benzyl benzoate, Benzyl Benzoate from Merck KGaA was used (quantitative value (assay) 100.3%, manufacturing lot No. K46888906 551).

### (Second step)

The benzyl benzoate (15 parts by mass) obtained through the first step (that is, purification treatment), 10 parts by mass of 96% by volume ethanol, 10 parts by mass of benzyl alcohol, and 5 parts by mass of fulvestrant were put in a clean glass container including a stirring rod, and mixed together by stirring, thereby obtaining a fulvestrant-containing solution.

The mixing time was 30 minutes, and the atmospheric temperature at the time of mixing was 25°C.

### (Third step)

Subsequently, castor oil was added to the fulvestrant-containing solution such that the total amount thereof was adjusted to 100 parts by mass, and the solution was homogenized by being further stirred, thereby obtaining a preparation for injection of Example C1.

For the obtained preparation for injection of Example C1, the rate of increase in fulvestrant sulfone was measured and evaluated by the same method as that described above.

As a result, it has been found that in the preparation for injection of Example C1, the rate of increase in fulvestrant sulfone was 4% which is lower than the rate of increase in fulvestrant sulfone in the preparation for injection of Comparative Example A2, and the increase in fulvestrant sulfone at the time of formulating the preparation for injection of Example C1 is suppressed.

As described above, it has been found that in a case where a preparation for injection is manufactured by performing at least one treatment selected from the group consisting of a treatment of mixing benzyl benzoate with an antioxidant, a treatment of heating benzyl benzoate, and a treatment of purifying benzyl benzoate, as claimed, and then mixing the treated benzyl benzoate with fulvestrant, the increase in fulvestrant sulfone at the time of formulating the preparation is suppressed.

## Claims

1. A method for manufacturing a preparation for injection, comprising:
a first step of performing at least one treatment selected from the group consisting of a treatment of mixing benzyl benzoate with an antioxidant, a treatment of heating benzyl benzoate, and a treatment of purifying benzyl benzoate; and
a second step of mixing the benzyl benzoate treated by the first step with fulvestrant,
wherein the antioxidant in the treatment of mixing benzyl benzoate with an antioxidant is at least one kind of compound selected from the group consisting of ascorbic acid, ascorbic acid palmitate, thiourea, α-tocopherol, δ-tocopherol, α-thioglycerin, cysteine hydrochloride, dihydrolipoic acid, dibutylhydroxytoluene, and propyl gallate,
wherein,
in the treatment of heating benzyl benzoate, benzyl benzoate is heated to 40 °C to 150°C in a nitrogen atmosphere, and
wherein,
in the treatment of purifying benzyl benzoate, benzyl benzoate is purified using a solid phase column.

2. The method for manufacturing a preparation for injection according to claim 1,
wherein the antioxidant in the treatment of mixing benzyl benzoate with an antioxidant is ascorbic acid.

3. The method for manufacturing a preparation for injection according to claim 1 or 2,
wherein an amount of the antioxidant in the treatment of mixing benzyl benzoate with an antioxidant is 0.1% by mass to 2% by mass with respect to an amount of benzyl benzoate.

4. The method for manufacturing a preparation for injection according to any one of claims 1 to 3,
wherein the treatment of mixing benzyl benzoate with an antioxidant is a treatment of mixing benzyl benzoate with an antioxidant and a medically acceptable solvent.

5. The method for manufacturing a preparation for injection according to claim 4,
wherein the medically acceptable solvent is an alcohol.

6. The method for manufacturing a preparation for injection according to claim 5,
wherein the alcohol is at least one alcohol selected from the group consisting of ethanol, benzyl alcohol, propylene glycol, polyethylene glycol, and glycofurol.

7. The method for manufacturing a preparation for injection according to claim 5 or 6,
wherein the alcohol is at least one alcohol selected from the group consisting of ethanol, benzyl alcohol, and propylene glycol.

## Patentansprüche

1. Verfahren zur Herstellung einer Injektionszubereitung, umfassend:
einen ersten Schritt des Durchführens mindestens einer Behandlung, ausgewählt aus der Gruppe bestehend aus einer Behandlung des Mischens von Benzylbenzoat mit einem Antioxidans, einer Behandlung des Erhitzens von Benzylbenzoat und einer Behandlung des Reinigens von Benzylbenzoat; und
einen zweiten Schritt des Mischens des im ersten Schritt behandelten Benzylbenzoats mit Fulvestrant,
wobei das Antioxidans bei der Behandlung des Mischens von Benzylbenzoat mit einem Antioxidans mindestens eine Art von Verbindung ist, die aus der Gruppe ausgewählt ist, die aus Ascorbinsäure, Ascorbinsäurepalmitat, Thioharnstoff, α-Tocopherol, δ-Tocopherol, α-Thioglycerin, Cysteinhydrochlorid, Dihydroliponsäure, Dibutylhydroxytoluol und Propylgallat besteht,
wobei bei der Behandlung des Erhitzens von Benzylbenzoat das Benzylbenzoat in einer Stickstoffatmosphäre auf 40°C bis 150°C erhitzt wird, und
wobei bei der Behandlung der Reinigung von Benzylbenzoat unter Verwendung einer Festphasensäule gereinigt wird.

2. Verfahren zur Herstellung einer Injektionszubereitung nach Anspruch 1,
wobei das Antioxidans bei der Behandlung des Mischens von Benzylbenzoat mit einem Antioxidans Ascorbinsäure ist.

3. Verfahren zur Herstellung einer Injektionszubereitung nach Anspruch 1 oder 2,
wobei die Menge des Antioxidans bei der Behandlung von des Mischens von Benzylbenzoat mit einem Antioxidans 0,1 Massenprozent bis 2 Massenprozent, bezogen auf die Menge an Benzylbenzoat, beträgt.

4. Verfahren zur Herstellung einer Injektionszubereitung nach einem der Ansprüche 1 bis 3,
wobei die Behandlung des Mischens von Benzylbenzoat mit einem Antioxidans eine Behandlung des Mischens von Benzylbenzoat mit einem Antioxidans und einem medizinisch akzeptablen Lösungsmittel ist.

5. Verfahren zur Herstellung einer Injektionszubereitung nach Anspruch 4,
wobei das medizinisch akzeptable Lösungsmittel ein Alkohol ist.

6. Verfahren zur Herstellung einer Injektionszubereitung nach Anspruch 5,
wobei der Alkohol mindestens ein Alkohol ist, der aus der Gruppe ausgewählt ist, die aus Ethanol, Benzylalkohol, Propylenglykol, Polyethylenglykol und Glycofurol besteht.

7. Verfahren zur Herstellung einer Injektionszubereitung nach Anspruch 5 oder 6,
wobei der Alkohol mindestens ein Alkohol aus der Gruppe bestehend aus Ethanol, Benzylalkohol und Propylenglykol ist.

## Revendications

1. Procédé destiné à fabriquer une préparation pour injection, comprenant les étapes suivantes :
une première étape pour réaliser au moins un traitement sélectionné parmi le groupe consistant en un traitement pour mélanger du benzoate de benzyle avec un antioxydant, un traitement pour chauffer du benzoate de benzyle, et un traitement pour purifier du benzoate de benzyle, et
une seconde étape pour mélanger le benzoate de benzyle traité lors de la première étape avec du fulvestrant,
dans lequel l'antioxydant dans le traitement pour mélanger le benzoate de benzyle avec un antioxydant est au moins un type de composé sélectionné parmi l'acide ascorbique, le palmitate d'acide ascorbique, la thio-urée, l'alpha-tocophérol, le δ-tocophérol, la α-thio-glycérine, le chlorhydrate de cystéine, l'acide dihydrolipoïque, le dibutylhydroxytoluène, et le gallate de propyle,
dans lequel lors du traitement pour chauffer du benzoate de benzyle, le benzoate de benzyle est chauffé à 40 °C jusqu'à 150 °C sous atmosphère d'azote, et
dans lequel lors du traitement pour purifier du benzoate de benzyle, le benzoate de benzyle est purifié à l'aide d'une colonne en phase solide.

2. Procédé destiné à fabriquer une préparation pour injection selon la revendication 1,
dans lequel l'antioxydant dans le traitement pour mélanger le benzoate de benzyle avec un antioxydant est l'acide ascorbique.

3. Procédé destiné à fabriquer une préparation pour injection selon la revendication 1 ou 2,
dans lequel une quantité de l'antioxydant dans le traitement pour mélanger le benzoate de benzyle avec un antioxydant s'étend de 0,1 % en masse à 2 % en masse par rapport à une quantité de benzoate de benzyle,

4. Procédé destiné à fabriquer une préparation pour injection selon l'une quelconque des revendications 1 à 3,
dans lequel le traitement pour mélanger le benzoate de benzyle avec un antioxydant est un traitement pour mélanger le benzoate de benzyle avec un antioxydant et un solvant acceptable sur le plan médical.

5. Procédé destiné à fabriquer une préparation pour injection selon la revendication 4, dans lequel le solvant acceptable sur le plan médical est un alcool.

6. Procédé destiné à fabriquer une préparation pour injection selon la revendication 5,
dans lequel l'alcool est au moins un alcool sélectionné parmi le groupe consistant en l'éthanol, l'alcool benzylique, le propylèneglycol, le polyéthylèneglycol, et le glycofurol.

7. Procédé destiné à fabriquer une préparation pour injection selon la revendication 5 ou 6,
dans lequel l'alcool est au moins un alcool sélectionné parmi le groupe consistant en l'éthanol, l'alcool benzylique, et le propylèneglycol.
